Europäisches Patentamt

(19) European Patent Office

Office européen des brevets

(11) Numéro de publication: **0 086 150 B1**

(12) **FASCICULE DE BREVET EUROPEEN**

(45) Date de publication du fascicule du brevet: 14.05.86

(21) Numéro de dépôt: 83400227.1

(22) Date de dépôt: 03.02.83

(51) Int. Cl.⁴: **C 04 B 35/52,** A 61 L 17/00, A 61 F 2/24, D 01 F 11/10

(54) Procédé pour la réalisation de parois déformables élastiquement en fibres de carbone.

(30) Priorité: 09.02.82 FR 8202094

(43) Date de publication de la demande: 17.08.83 Bulletin 83/33

(45) Mention de la délivrance du brevet: 14.05.86 Bulletin 86/20

(84) Etats contractants désignés: CH DE GB IT LI NL

(56) Documents cités:
FR - A - 1 147 980
FR - A - 1 461 567
FR - A - 1 500 461
FR - A - 2 399 237
FR - A - 2 427 197
US - A - 3 991 248

(73) Titulaire: **SOCIETE EUROPEENNE DE PROPULSION (S.E.P.) Société Anonyme dite:, 3, avenue du Général de Gaulle, F-92800 Puteaux (FR)**

(72) Inventeur: **Buttazzoni, Bernard, "Le Galion" 23 Bld. A.Cieussa, F-13007 Marseille (FR)**

(74) Mandataire: **Hasenrader, Hubert, Cabinet BEAU DE LOMENIE 55, rue d'Amsterdam, F-75008 Paris (FR)**

## Description

L'invention concerne un procédé de réalisation de parois en fibres de carbone déformables élastiquement par rapport à une forme d'équilibre.

La Demanderesse a déjà fait connaître par le document FR-A 2 427 197 un procédé de fabrication de pièces composites à base de fibres de carbone. Ce procédé comporte le traitement d'un empilage de strates en fibres de carbone par infiltration de pyrocarbone, visant à densifier la structure, à lier structurellement la texture fibreuse par le pyrocarbone, en vue d'obtenir un produit final complétement massif, rigide et indéformable, destiné à être usiné pour les applications ultérieures.

De même, on connaît par le document FR-A 3 991 248 un traitement d'infiltration semblable visant à lier le substrat fibreux par du carbone pyrolitique puis à en achever la densification, pour obtenir un produit final solide, notamment utilisé dans les dispositifs de freinage puissant par friction.

Au contraire de ces traitements connus, la présente invention vise à réaliser, à partir d'un substrat de fibres de carbone, des parois capables de subir des déformations et de revenir à une position d'équilibre par rappel élastique.

Selon l'invention, pour fabriquer de telles parois, on utilise un substrat ou texture souple de fibres de carbone, étant entendu qu'on appelle ici texture souple l'ensemble d'une ou plusieurs couches indépendantes de parois réalisées par tout procédé textile connu (tissage, tressage, tricotage, feutrage, aiguilletage, etc.); on impose à cette texture une forme correspondant à la position d'équilibre voulue et on empèse la texture dans cette forme, par formation autour de chaque fibre d'une gaine mince de carbone pyrolitique infiltré en profondeur en phase vapeur à haute température, cette gaine ne créant pas de pontages aux jonctions interfibres de la texture. La formation d'une mince pellicule autour de chaque fibre de la texture donne à l'ensemble une élasticité qui permet à la structure de revenir à sa forme d'équilibre au repos par rappel élastique après avoir été déformée sous l'effet d'une sollicitation quelconque.

La texture utilisée peut être une gaine tressée, comprimée axialement ou au contraire étirée axialement pour lui donner sa forme d'équilibre une fois figée. Une telle texture permet la réalisation de tubes ou conduits souples et extensibles, destinés à être fixés par leurs deux extrémités à deux embouts dont la position respective est variable dans l'espace dans des limites données tout en assurant l'écoulement d'un fluide sans perturbations dans toutes les positions prises par les embouts.

De nombreux dispositifs existent sur le marché, soit sous forme de conduits plastiques ou à base de caoutchouc, soit sous forme de textures tissées ou tressées à partir de polymères, ces textures pouvant être armées par un renfort métallique enrubané.

En général, ces conduits ne sont pas extensibles longitudinalement et ne supportent pas un désaxage ou un coude trop prononcé sans s'aplatir ou se détériorer.

Leur tenue en température, limitée à celle des matériaux constitutifs, ne dépasse pas de ce fait quelques centaines de degrés.

Selon un des aspects de l'invention, on peut réaliser des conduits extensibles longitudinalement, résistant à l'aplatissement et tenant à des températures supérieures à 1000 °C.

De façon avantageuse et non limitative, les textures obtenues en fibres de carbone traitées au pyrocarbone donnent des structures biocompatibles utilisables comme prothèses et notamment comme prothèses vasculaires et renforcements cutanés ou cartilagineux.

Il est à noter que le document FR-A 2 399 237 fait connaître des prothèses vasculaires réalisées en étoffe dont les fibres sont revêtues de carbone. Les fibres de l'étoffe ne sont pas en carbone, mais des fibres d'organopolymère. Le revêtement de carbone n'est là que pour assurer la biocompatibilité de la prothèse; les propriétés physiques, et notamment la flexibilité ou l'élasticité du produit final, sont celles de l'étoffe d'origine, au contraire de la présente invention.

D'autres caractéristiques et avantages de l'invention ressortiront de la description suivante de plusiuers réalisations préférentielles, faite en référence aux dessins annexés sur lesquels:

– la figure 1 représente une gaine de fibres tressée avant traitement,

– les figures 2 et 3 représentent le maintien à la forme d'équilibre de la texture de la figure 1, selon deux modes de réalisation correspondant à des taux de compression de la texture différents,

– les figures 4 et 5 représentent les textures des figures 2 et 3 au repos (en traits pleins) et en extension sous une force de 100 g (en traits pointillés);

– les figures 6 et 7 représentent les textures des figures 2 et 3 en position coudée;

– les figures 8 et 9 représentent un autre mode de réalisation d'une texture conforme à l'invention, une valve cardiaque, respectivement fermée dans sa forme de repos, et ouverte sous l'influence d'un écoulement;

– les figures 10 et 11 représentent une variante de la valve cardiaque des figures 8 et 9.

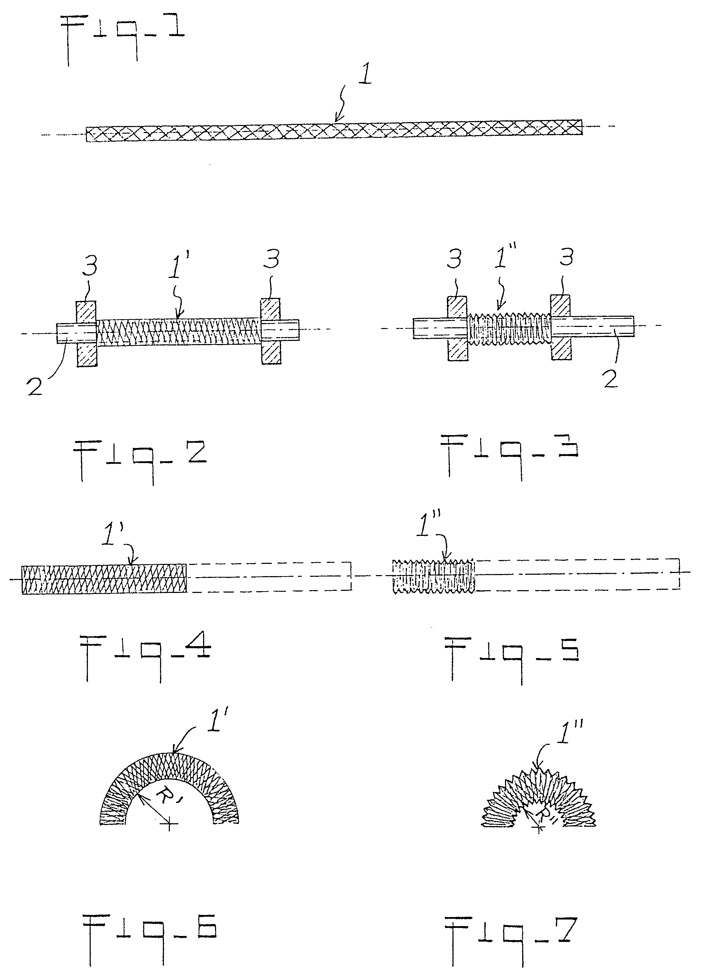

Les figures 1 à 7 montrent des réalisations de conduits vasculaires en fibres de carbone qui possèdent des propriétés optimales d'hémocompatibilité.

Sur la figure 1 est représentée une gaine 1 tressée avec 32 mèches de 1000 filaments sur un diamètre intérieur de 3 mm. Le tronçon de gaine brute fait 120 mm.

Afin de lui donner, par compression, la forme d'équilibre désirée, on enfile la gaine sur un mandrin 2 de graphite, au moins partiellement fileté, par exemple à ses deux extrémités pour y visser des écrous de maintien 3 permettant de régler le taux de compression choisi.

Selon la figure 2, la gaine est comprimée à un taux de 1/3 (rapport longueur comprimée sur longueur brute) sur un diamètre de 5 mm.

On fige cette forme comprimée par infiltration de l'ensemble par du pyrocarbone en phase vapeur à haute température selon les techniques connues du dépôt chimique en phase vapeur.

Le dosage du traitement est étudié de manière à obtenir autour de chaque filament élémentaire de carbone (de l'ordre de 8 µm de diamètre) un film de pyrocarbone d'épaisseur comprise entre 0,2 et 1,5 µm. Ce film de pyrocarbone fige chaque filament dans sa forme maintenue sur l'outillage et lui procure une plus ou moins grande flexibilité selon son épaisseur.

Une fois extrait de son mandrin (figure 4) le conduit 1′ conserve la forme imposée par l'outillage, mais il est capable de subir des déformations de grande amplitude et de revenir à sa forme d'équilibre sans altération.

La figure 4 montre le tube libre à spires jointives obtenu, dont la raideur d'extensibilité longitudinale varie selon le traitement, et peut par exemple atteindre 120% d'allongement sous 100 grammes force.

A 100% d'allongement le rétrécissement diamétral est de 25%. La pression d'écrasement est de l'ordre de 0,5 bar. Ce tube libre flexible est insensible à l'effet d'écrasement ou de pliure sous de faibles rayons de courbure (allant jusqu'à R′ = deux fois le diamètre, figure 6) ou sous une déformation en torsion.

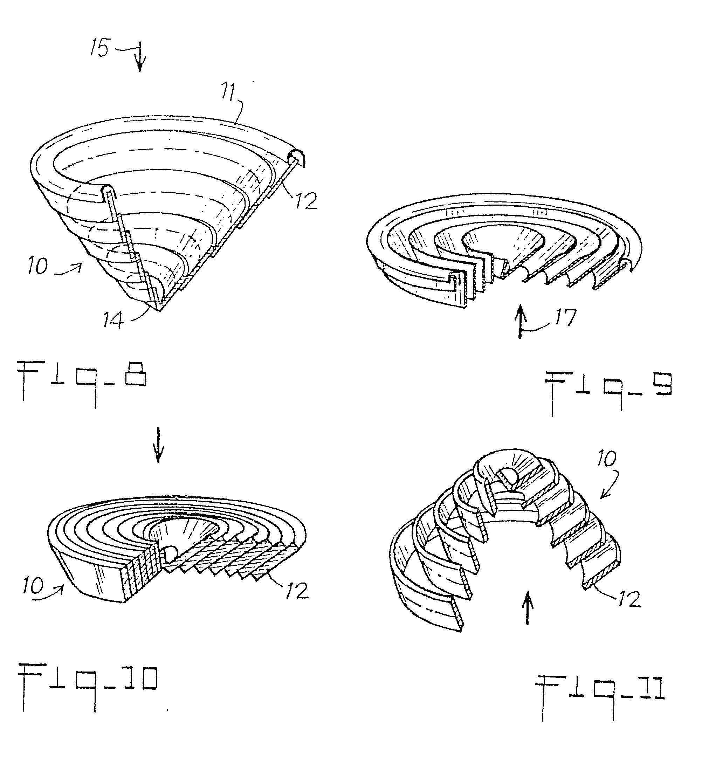

En prenant un taux de compression de 1/6 (figure 3), on va jusqu'à faire «flamber» la texture tressée, et on obtient un tube ondulé en accordéon 1″ dont la raideur d'élongation exiale est encore plus faible (par exemple 300% d'allongement sous 100 g force, figure 5). A 100% d'allongement, le rétrécissement diamétral est de 20% et la pression d'écrasement supérieure à 1 bar. Le tube peut supporter sans écrasement d'être coudé avec un rayon R″ de courbure égal au diamètre du tube.

Un avantage du procédé de l'invention en chirurgie vasculaire est de pouvoir obtenir, avec du carbone pur, des conduits flexibles que l'on ne peut pas réaliser par tissage ou tricotage direct, soit avec des fibres polymères, soit avec des fibres de carbone.

La texture comprimée dans sa position au repos offre une compacité suffisante pour que la porosité résiduelle soit traitée par les moyens habituels utilisés avec les textures polymères.

Le procédé de fabrication est applicable à tous diamètres de gaine et à des formes géométriques qui ne sont pas nécessairement de révolution. On peut obtenir ainsi des conduits de 16 mm de diamètre pour des prothèses trachéales comportant un méplat. En retroussant les extrémités de la gaine sur elle-même, les bords de la prothèse ne s'effilochent pas.

Selon un autre mode de réalisation particulièrement avantageux, on peut fabriquer des valves anti-retour, notamment des valves cardiaques.

La figure 8 montre une valve anti-retour 10 en position de repos, comprenant une couronne 11 à l'intérieur de laquelle un ruban continu souple 12 s'enroule en spires tronconiques successives.

Dans cette position de repos, le ruban forme donc une pseudo-membrane étanche, de forme conique, ayant, dans un exemple précis de réalisation, 25 mm de diamètre à la base et 18 mm de hauteur, la couronne faisant quant à elle 5 mm de hauteur.

Chaque spire tronconique recouvre partiellement la précédente qui lui est intérieure. Le recouvrement se fait dans le sens de la base du cône vers le sommet par appui de laspire la plus petite sur celle du tour suivant et ainsi de suite, en commençant l'enroulement par la spire centrale 14, laquelle est conformée en cône (de façon à assurer l'étanchéité à son niveau). Ce cône peut être percé d'un orifice calibré pour assurer une fuite en retour contrôlée.

La valve 10 garde la position fermée représentée sur la figure 8 tant que la différence de pression du fluide s'exerce dans le sens de la flèche 15, ou encore lorsque cette différence de pression est nulle.

Lorsque la différence de pression du fluide s'exerce dans l'autre sens, comme représenté par la flèche 17 de la figure 9, la valve 10 passe, sous l'effet de cette pression, dans une seconde position résultant d'un équilibre entre la force de rappel élastique des spires du ruban vers la première position et l'effort de pression dynamique du fluide.

Pour former cette valve le ruban générateur de la pseudo-spirale est:

– soit découpé dans la texture à plat dans son plan,

– soit obtenu directement par tissage spécial en hélice à plat à diamètre évolutif,

– soit construit à partir de secteurs angulaires préalablement découpés dans le plan puis raccordés suivant une génératrice, l'ensemble constituant l'hélice précédente.

Le formage de la pseudo-spirale conique se fait sur outillage conique en commençant par le centre conique plein. La forme développée et l'angle du cône sont adaptés pour qu'une fois l'enroulement terminé, les génératrices au droit du recouvrement des spires soient parallèles.

Le formage de la couronne circulaire ne présente pas de difficulté particulière en utilisant ici également un ruban de tissu de carbone ourlé et cintré au diamètre voulu sur outillage.

L'intégration de la couronne sur le cône se fait par recouvrement de la dernière spire sur l'ourlet intérieur, comme on le voit sur la figure 8. Les deux éléments peuvent être réunis par une couture en fil de carbone ou un scellement au carbone.

Le traitement du tissu au pyrocarbone intervient ensuite pour lui donner la raideur élastique voulue: un outillage réfractaire adéquat ou une couture provisoire avec un fil d'un matériau s'éliminant à haute température, permet de maintenir la pseudospirale en position fermée, les spires étant en contact au droit de leur recouvrement.

Ce traitement se fait à haute température (au-dessus de 900 °C) par infiltration de pyrocarbone en phase vapeur dans la trame même de la texture. Cet «empesage» au carbone associé à la nature même des fibres de carbone, fige la pseudo-spirale dans une position de repos stable qui, dès qu'on tente de l'en écarter par déplacement axial du sommet vers le base, entraîne un effort de rappel induit par la contrainte de flexion/torsion qui se manifeste aussitôt dans la section courante transversale du ruban spiralé.

Les performances d'une telle valve sont en mesure de satisfaire les conditions de fonctionnement d'une valve naturelle. Dans un exemple particulier de réalisation, il a été mesuré les caractéristiques suivantes:

– tenue de la pseudo-spirale à la pression retour: supérieure à 250 mm Hg, valeur spécifiée pour les valves biologiques

– surpression d'ouverture inférieure à 10 mm Hg spécifiés

– fuite avec valve brute de traitement: 20 cm³/s d'eau sous 25 mm Hg

–débit à pleine ouverture supérieur à 500 cm³/s d'eau sous 25 mm Hg

– déplacement total du sommet à plein débit: 20 mm

– fréquence propre dans l'air sensiblement égale à 10 Hz

– masse de la spirale: 0,4 g et diamètre $\emptyset$ 25 mm

– très grande résistance à la fatigue tant que les déformations restent modérées.

Le conditionnement préalable de la valve juste avant implantation, s'opère selon le procédé connu de l'imperméabilisation de la texture des spires par coagulation sanguine pré-opératoire.

L'implantation consiste à insérer dans le sens voulu la valve conique munie de sa couronne perpendiculairement dans le conduit aortique sans se soucier de l'orientation angulaire, puis à suturer à travers les mailles du tissu constitutif la couronne et la paroi vasculaire à l'endroit désiré.

Le fonctionnement en circulation cardiaque normale repose sur la propriété des matériaux carbonés de se recouvrir d'un néotissu de 100 µm au plus d'épaisseur finale au bout de deux mois lorsque la texture est baignée par le flux sanguin. Ce néotissu achève l'imperméabilisation de la texture et crée un état de surface homogène favorisant l'étanchéité au droit du recouvrement des spires. Le battement permanent des spires avec un écartement de plus de 3 mm empêche la formation de thrombose.

Par ailleurs, une colonisation tissulaire intervient au niveau de la couronne contre la paroi vasculaire.

Selon le type de valve – mitrale ou tricuspide – des architectures différentes peuvent être nécessaires. Une variante peut ainsi se réaliser à partir d'une forme au repos plane (figures 10 et 11) donc moins encombrante dans le sens amont (valve dite «plate»).

Dans ce cas, les spires tronconiques, à largeur évolutive ou constante, sont en appui à recouvrement total de façon que leur bordure amont et/ou aval, reste dans un même plan, quand la valve est au repos.

Une autre application de l'invention pour des valves cardiaques consiste à partir d'une forme tubulaire en tissu de carbone, dont on rapproche le bord sous forme de trois lèvres, l'élasticité des lèvres étant assurée par le traitement au pyrocarbone.

Un autre type d'application du procédé de l'invention à une texture en forme de gaine peut se faire en maintenant la gaine en extension sur son mandrin: la tresse obtenue offre alors une grande raideur d'allongement et devient un élément de renfort tendineux ou ligamentaire ayant une élasticité de 10% à rupture au lieu d'une élasticité nulle avec des fibres uniquement longitudinales.

Selon encore une autre application de l'invention, on part d'une texture plane en tissu «toile» de carbone, qu'on fige dans cette configuration en la maintenant entre des plateaux de graphite et en la traitant au pyrocarbone. On obtient ainsi une paroi plane à laquelle est conférée un rappel élastique, dès déformation, d'autant plus important que le rayon de cintrage de la texture déformée est plus grand. La texture obtenue peut notamment être utilisée pour le renforcement cartilagineux des parois auriculaires en chirurgie vétérinaire pour redressement des oreilles de chien.

Comme on l'a compris, le principe de l'invention repose sur le dépôt d'un film mince, d'épaisseur comprise entre 0,2 micron (µm) et 1,5 micron (µm) selon la flexibilité désirée, avec une valeur en moyenne de 0,5 micron (µm).

En raison de la minceur de cette gaine de pyrocarbone, le traitement de dépôt n'intéresse que le pourtour des fibres élémentaires de carbone (de l'ordre de 8 microns (µm) de diamètre) et n'affecte pas les jonctions interfibres de la texture de départ par des pontages pyrocarbone qui risqueraient de rigidifier l'ensemble.

**Revendications**

1. Procédé de réalisation de parois en fibres de carbone, capables de subir des déformations et de revenir à une position d'équilibre par rappel élastique, caractérisé en ce qu'on utilise une texture de départ souple (1, 12) de fibres de carbone, on impose à cette texture une forme correspondant à la position d'équilibre (1', 1'', 10) recherchée, et on empèse la texture, dans cette forme, par formation autour de chaque fibre d'une gaine mince de préférence de 0,2 à 1,5 microns (µm) d'épaisseur de carbone pyrolitique infiltré en profondeur en phase vapeur à haute température, cette gaine ne créant pas de pontages aux jonctions interfibres de la texture, moyennant quoi l'empesage de pyrocarbone confère à la texture une flexibilité élastique se traduisant par un rappel élastique vers la position d'équilibre dès qu'on écarte la texture de cette position.

2. Procédé selon la revendication 1, caractérisé en ce qu'on impose à la texture (1, 12) sa forme d'équilibre (1', 1'', 10) en la maintenant contre la surface d'un outil (2) correspondant à ladite forme.

3. Procédé selon la revendication 2, caractérisé en ce qu'on impose à la texture une forme plane par utilisation de plateaux de maintien.

4. Procédé selon la revendication 1, caractérisé en ce qu'on impose à la texture sa forme d'équilibre par couture à l'aide d'un fil disparaissant à haute température.

5. Procédé selon l'une quelconque des revendications 1 à 4, caractérisé en ce qu'on utilise comme texture une gaine tressée (1) qu'on comprime axialement pour lui imposer sa forme d'équilibre (1', 1'').

6. Procédé selon l'une quelconque des revendications 1 à 4, caractérisé en ce qu'on utilise comme texture une gaine tressée (1) qu'on maintient en extension axiale pour lui imposer sa forme d'équilibre.

7. Procédé selon l'une quelconque des revendications 5 ou 6, caractérisé en ce qu'on place la gaine tressée (1) autour d'un mandrin (2) pour lui imposer sa forme d'équilibre.

8. Procédé selon l'une quelconque des revendications 1 à 4, caractérisé en ce qu'on utilise comme texture un ruban tissé (12) qu'on enroule en pseudospirale conique (10) pour lui donner sa forme d'équilibre.

## Patentansprüche

1. Verfahren zur Herstellung verformbarer Wandungen aus Kohlenstoffasern, die über elastische Rückstellkräfte in eine Gleichgewichtslage zurückfedern, dadurch gekennzeichnet, dass eine weiche Ausgangstextur (1, 12) aus Kohlenstofffasern verwendet wird, dieser Textur eine der gewünschten Gleichgewichtslage (1', 1'', 10) entsprechende Form gegeben wird und die Textur dieser Form versteift wird, in dem um jede Faser eine dünne Schutzschicht, vorzugsweise mit einer Dicke zwischen 0,2 bis 1,5 µm aus pyrolitischem Kohlenstoff gebildet wird, die bei hoher Temperatur in der Dampfphase tief infiltriert wird, wobei diese Schutzschicht keine Überbrückungen an den Verbindungspunkten zwischen den Fasern der Textur bildet, wodurch die Pyrokohlenstoffversteifung der Textur eine elastische Flexibilität verleiht, die zu einer elastischen Rückstellung in die Gleichgewichtslage führt, wenn die Textur aus dieser Lage versetzt wird.

2. Verfahren nach Anspruch 1, dadurch gekennzeichnet, dass der Textur (1, 12) ihre Gleichgewichtsform (1', 1'', 10) gegeben wird, in dem sie gegen die Oberfläche eines der Form entsprechenden Werkzeugs (2) angedrückt wird.

3. Verfahren nach Anspruch 2, dadurch gekennzeichnet, dass der Textur eine ebene Form gegeben wird, indem Halteplatten verwendet werden.

4. Verfahren nach Anspruch 1, dadurch gekennzeichnet, dass der Textur ihre Gleichgewichtsform gegeben wird, indem sie mittels eines sich bei hoher Temperatur auflösenden Fadens vernäht wird.

5. Verfahren nach einem der Ansprüche 1 bis 4, dadurch gekennzeichnet, dass als Textur eine geflochtene Hülse (1) verwendet wird, die axial komprimiert wird, um ihr ihre Gleichgewichtsform (1', 1'') zu geben.

6. Verfahren nach einem der Ansprüche 1 bis 5, dadurch verwendet, dass als Textur eine geflochtene Hülse (1) verwendet wird, die axial gestreckt wird, um ihr ihre Gleichgewichtsform zu geben.

7. Verfahren nach einem der Ansprüche 5 oder 6, dadurch gekennzeichnet, dass die geflochtene Hülse (1) um eine Wickelhülse (2) gewickelt wird, um ihr ihre Gleichgewichtsform zu geben.

8. Verfahren nach einem der Ansprüche 1 bis 4, dadurch gekennzeichnet, dass als Textur ein gewebtes Band (12) verwendet wird, das in Form einer konischen Pseudospirale (10) aufgerollt wird, um ihr ihre Gleichgewichtsform zu geben.

## Claims

1. Method for producing walls in carbon fibers, capable of withstanding deformations and of elastically returning to an at rest shape, characterized in that a flexible starting texture (1, 12) of carbon fibers is used, a shape corresponding to the at rest shape (1', 1'', 10) desired is imposed to said texture, and the texture is fixed in said shape by forming around said fibers a thin sheath, preferably between 0.2 to 1.5 µm thickness of pyrolytic carbon deeply infiltrated by chemical vapors at high temperature, said sheath creating no divisions at interfiber junctions of the texture, as a result of which the pyrocarbon stiffening gives to the structure an elastical flexibility, resulting in an elastic return to the at rest shape, as soon as the texture is moved from that position.

2. Method according to claim 1, characterized in that the at rest shape (1', 1'', 10) is given to the texture (1, 12) by holding the latter against the surface of a tool (2) corresponding to said imposed form.

3. Method according to claim 2, characterized in that the texture is given a flat shape by way of holding plates.

4. Method according to claim 1, characterized in that the texture is given its at rest shape by way of a seam using a thread which dissolves at high temperature.

5. Method according to any one of claims 1 to 4, characterized in that the texture used is a braided sleeve (1) which is compressed axially to give it its at rest shape (1', 1'').

6. Method according to any one of claims 1 to 4, characterized in that the texture used is a braided sleeve (1) which is held axially stretched to give it its at rest shape.

7. Method according to any one of claims 5 or 6, characterized in that the braided sheath (1) is placed over a mandrel (2) to give it its at rest shape.

8. Method according to any one of claims 1 to 4, characterized in that the texture used is a woven tape (12) which is wound in conical pseudo-spiral (10) to give it its at rest shape.

Fig-1

Fig-2

Fig-3

Fig-4

Fig-5

Fig-6

Fig-7

Fig. 8

Fig. 9

Fig. 10

Fig. 11